(19) Europäisches Patentamt

European Patent Office

Office européen des brevets

(11) Veröffentlichungsnummer: **0 412 507 A1**

## (12) EUROPÄISCHE PATENTANMELDUNG

(21) Anmeldenummer: 90115168.8

(22) Anmeldetag: 07.08.90

(51) Int. Cl.⁵: **B01D 69/02**

(30) Priorität: 08.08.89 DD 331588
08.08.89 DD 331589
08.08.89 DD 331587

(43) Veröffentlichungstag der Anmeldung:
**13.02.91 Patentblatt 91/07**

(84) Benannte Vertragsstaaten:
**CH DE FR GB LI NL SE**

(71) Anmelder: **Humboldt-Universität zu Berlin**
**Unter den Linden 6**
**DDR-1086 Berlin(DD)**

(72) Erfinder: **Ehwald, Rudolf, Dr.sc.nat., Dipl.-Biol.**
**Egon-Schulz-Strasse 56**
**DD-1040 Berlin(DD)**
Erfinder: **Klein, Ulrich, Dr.rer.nat., Dipl.-Chem.**
**Erich-Weinert-Strasse 116**
**DD-1055 Berlin(DD)**
Erfinder: **Jäschke, Andreas, Dipl.-Chem.**
**Delbrückstrasse 5**
**DD-1136 Berlin(DD)**
Erfinder: **Cech, Dieter, Dr.sc.nat. Dipl.-Chem.**
**Leninallee 216**
**DD-1156 Berlin(DD)**
Erfinder: **Titel, Christine, Dr.rer.nat. Dipl.-Biol.**
**Mühlenweg 18**
**DD-1401 Nassenheide(DD)**

(74) Vertreter: **Spott, Gottfried, Dr.**
**Spott Weinmiller & Partner**
**Sendlinger-Tor-Platz 11**
**D-8000 München 2(DE)**

㉝ Verfahren zur Herstellung von Hohlträgern mit eingeschlossenen diffusionsfähigen hochmolekularen Stoffen mit möglicher Grössenfraktionierung von Makromolekülen.

㉝ Das Verfahren betrifft den Einschluß von Makromolekülen in präformierte partikuläre Hohlmembransysteme (vesikuläre Träger bzw. Mikrokapseln) und ihre fraktionierte Freisetzung. Die Einführung der Makromoleküle in die Hohlträger erfolgt durch Verwendung eines permeationsvermittelnden Dispersionsmittels. Als solches wird eine flüssige Phase eingesetzt, in der die Molekülgröße der einzuschließenden Makromoleküle unter ihrem Maximum sowie unter der Molekülgrößen-Trenngrenze der Hohlträgermembran liegt. Anschließend wird das Dispersionsmittel unter Ausnutzung bekannter Gesetzmäßigkeiten verändert oder ausgetauscht, wodurch sich die effektive Teilchengröße der Makromoleküle im Verhältnis zur Trenngrenze der Hohlträgermembran vergrößert und sie überschreitet. Die fraktionierte Freisetzung der eingeschlossenen Makromoleküle durch Anwendung zunehmend permeabilitätsvermittelnder Elutionsflüssigkeiten wird zur Größenfraktionierung polydisperser Polymerengemische eingesetzt.

EP 0 412 507 A1

## VERFAHREN ZUR HERSTELLUNG VON HOHLTRÄGERN MIT EINGESCHLOSSENEN DIFFUSIONSFÄHIGEN HOCHMOLEKULAREN STOFFEN MIT MÖGLICHER GRÖSSENFRAKTIONIERUNG VON MAKROMOLEKÜLEN

Die Erfindung betrifft den Einschluß von Makromolekülen in Hohlträger bzw. Mikrokapseln mit geschlossenen semipermeablen Hüllmembranen und eine mit diesem Einschluß mögliche Größenfraktionierung der Makromoleküle . Die Erfindung kann in der Pharmazie und Biotechnologie eingesetzt werden.

Der Einschluß von Makromolekülen in Hohlträger hat gegenüber anderen Verfahren der Immobilisierung den Vorteil der Unabhängigkeit der Konzentration des immobilisierten Stoffes von den Eigenschaften der Festphase und der Diffusionsfähigkeit der eingeschlossenen Kolloide im Trägerinnenraum (Hartmeier, V. Immobilisierte Biokatalysatoren. Springer, Berlin, Heidelberg, New York, Tokyo, 1986). Begrenzungen des Enkapsulationsprinzipes bestehen in oft ungenügender Zugänglichkeit der immobilisierten Makromoleküle für die Diffusion von Substraten oder Linganden, ungenügender hydrodynamischer Flußrate im Festbett und chemischer Veränderung der Makromoleküle mit den für die Kapselbildung eingesetzten Reaktionskomponenten. Der Einschluß von Makromolekülen in vorgefertigte Hohlträger mit geschlossener Hüllmembran ist bekanntlich (US 4 257 884) durch eine irreversible chemische Veränderung der Hüllmembran (Vernetzung, Beschichtung mit gleichzeitiger Erniedrigung der Permeabilitätsgrenze der Membran) möglich. Hinsichtlich der Reaktionsmöglichkeit der einzuschließenden Makromoleküle mit den zur Beschichtung bzw. Veränderung der Membran benutzten Reagenzien bestehen ähnliche Einschränkungen wie bei Einschluß durch Kapselneubildung. Hohlfasern und andere offene Hohlmembranmodule haben den Vorteil, daß der innere Hohlraum verlustfrei mit den einzuschließenden Makromolekülen gefüllt werden kann. In den bekannten Hohlmembranmodulen ist allerdings die Geschwindigkeit der Substratpermeation begrenzt. Wegen der genannten Probleme besteht ein begründetes technisches Interesse an einem ökonomischen Verfahren zum Einschluß von Makromolekülen in preiswerte Hohlträger mit geschlossenen semipermeablen Hüllmembranen, die durch eine hohe Stoffaustauschrate für alle permeablen Stoffe ausgezeichnet sind. Sehr hohe spezifische Austauschraten besitzen Mikrokapseln mit sehr dünnen Hüllmembranen, z.B. vesikuläre Träger gemäß DD 247 570 A3. Bekannte Verfahren für den Einschluß von Makromolekülen in derartige Hohlträger erfordern eine chemische Veränderung (Aggregation, Polymerisation) permeabler Moleküle. Vesikuläre Träger besitzen Ultrafiltermembranen und sind für die Größenfraktionierung von Makromolekülen geeignet. Grundsätzlich ermöglichen die bekannten Membrantrennverfahren mit einer Membran lediglich die Gewinnung von zwei Fraktionen - des Permeats und des Retentats. Soll eine Auftrennung der Probe in mehr als zwei Fraktionen erreicht werden, dann müssen Permeat oder Retentat an Membranen mit anderen Trennbereichen nochmals fraktioniert werden. Dies trifft auch sinngemäß für die Vesikelchromatographie gemäß DD 247 570 A3 und DD 268 744 A3 zu. Resultat einer chromatographischen Trennung mit vesikulären Trägern sind eine ausgeschlossene und eine permeierende Fraktion. Mehr als zwei Größenfraktionen können nur durch hintereinander geschaltete Trennungen mit unterschiedlichem Trennsystem bzw. unterschiedlicher Molmassentrenngrenze gewonnen werden.

Der Erfindung liegt die Aufgabe zugrunde, Makromoleküle durch die Hüllmembran eines präformierten geschossenen semipermeablen Hohlmembransystems, z.B. von vesikulären Trägern, in die stationäre flüssige Phase des Trägerhohlraums einzuführen und dort in Abhängigkeit von ihrer Größe mit der Möglichkeit der fraktionierten Freisetzung reversibel einzuschließen, ohne die Makromoleküle oder die Hüllmembran irreversibel zu verändern.

Die Aufgabe wird dadurch gelöst, daß Hohlträger, umhüllt durch eine Ultrafiltermembran mit scharfer Permeabilitätsgrenze, mit einer Dispersion einzuschließender hochmolekularer Stoffe, deren effektiver Moleküldurchmesser in Abhängigkeit vom Dispersionsmittel unter oder über der Molekülgrößentrenngrenze der Hohlträgermembran liegt, versetzt werden. Hierzu wird zuerst unter Ausnutzung bekannter Gesetzmäßigkeiten der Dispersionsmittelabhängigkeit der effektiven Molekülgröße polymerer Stoffe bzw. der Molmassen-Trenngrenze der Hohlträgermembran ein permeabilitätsvermittelndes Dispersionsmittel gewählt, in dem die einzuschließenden makromolekularen Stoffe durch die Hohlträgermembran permeieren können. Danach werden die effektiven Moleküldurchmesser der einzuschließenden Stoffe über den Wert der Molekülgrößen-Permeabilitätsgrenze der Hohlträgermembran vergrößert. Dies geschieht durch Veränderung der Zusammensetzung des flüssigen Dispersionsmittels, grundsätzlich durch Zusatz von Stoffen, die die Molmassen-Permeabilitätsgrenze der Makromoleküle für die Hohlträgermembran verringern oder durch Herabsetzung der Konzentration solcher Stoffe, die das Molmassen-Permeabilitätslimit vergrößern, oder aber durch vollständigen Austausch des Dispersionsmittels. Dabei werden in der Polymerenchemie grundsätzlich bekannte Gesetzmäßigkeiten der Abhängigkeit der Molekülgröße und -gestalt bzw. des Molmassen-Permeabklitätslimits der Membran vom Dispersionsmittel ausgenutzt (Autorenkollektiv, Strukturuntersuchungen an Biopolymeren mit spektroskopischen und hydrodynamischen Methoden, Akademie-Verlag, Berlin, 1976, Patent DD 269 744 A3). Das Ausmaß der dispersionsmittelabhängigen Variabilität des Molmassen-Permeabilitätslimits hydrophiler Makromoleküle, u.a. löslicher Polysaccharide und Nucleinsäuren, an dünnen Ultrafiltermembranen ist bei bestimmten erfindungsgemäßen Dispersionsmittelveränderungen unerwartet hoch und kann daher effektiv für den Einschluß in Hohlträger mit geschlossener Hüllmembran effektiv genutzt werden. Die Erfindung ist nicht auf die Verwendung von zellstrukturierten bzw. vesikulären Trägern, in denen eine Zellwandstruktur trennwirksam ist, beschränkt, da sich gezeigt hat, daß der Einfluß verschiedener Komponenten des Dispersionsmittels auf die Permeabilität dünner Ultrafiltermembranen für bestimmte polymere Stoffe, z.B. der Einfluß hoher Ionenstärken auf die maximale Molmasse für die Permeabilität der Polyelektrolyte und der Einfluß hoher Alkohlkonzentrationen auf die maximale Molmasse für de Permeabilität der Dextranmoleküle, unabhängig von der Art der Membran auftritt. Besonders geeignet für den erfindungsgemäßen Einschluß in präformierte geschlossene Hohlträger sind hydrophile polymere Stoffe mit stark hydratationsabhängiger Molekulargröße wir Dextrane, weitere wasserlösliche neutrale oder saure Kohlenhydrate, Nukleinsäuren, Polyethylenglycole, Polyvinylpyrrolidon, Polyacrylamid und Polyacrylsäure. Anstelle von einfachen polymeren Stoffen können auch deren Derivate oder zusammengesetzte polymere Stoffe mit kupplungsfähigen funktionellen Gruppen für die Bindung von Enzymen oder Wirkstoffen oder mit Rezeptoren für die Affinitätsbindung, z.B. mit Periodat oxydiertes Dextran oder Verbindungen von Dextran mit Cibacromblau F3GA (blue Dextran), erfindungsgemaäß eingeschlossen werden.

Der Zusatz von Ethanol, Aceton oder ähnlichen wasserlöslichen organischen Stoffen zum Dispersionsmittel bis zu einer Konzentration wenig unterhalb des für die Fällung erforderlichen Wertes führt an dünnen aus Pflanzenzellwänden hergestellten Ultrafiltermembranen zu einer beträchtlichen Vergrößerung der Molmassengrenze für den Membrandurchtritt von Makromolekülen mit flexibler, hydratationsabhängiger Molekülgröße. Dieser Effekt ist überrachenderweise im Fall der Dextranmoleküle bei niedrigen Salzkonzentrationen wesentlich stärker als bei hohen Salzkonzentrationen. Während in Wasser oder wäßrigen Salzlösungen das Molmassen-Permeabilitätslimit der Dextrane für die aus einer pflanzlichen Zellwand bestehenden Ultrafiltermembranen des vesikulären Trägermaterials bei etwa 15 kD liegt, permeiiert in 35 %igem salzfreiem wäßrigen Ethanol bereits etwa die Hälfte des Hochmolekularen polydispersen Dextranpräparats Dextran 250 (mitlere Molmasse bei 250 kD) in den Innenraum der Träger. Bei höheren Ionenstärken (z.B. 0,1 M Phosphatpuffer) und gleichem Ethanolgehalt ist dagegen der größte Teil des Dextranpräparats unpermeabel. Für den Einschluß von Dextranmolekülen in aus Pflanzenzellen hergestellte vesikuläre Träger ist es daher vorteilhaft, Dextanpräparate mit Molmassen deutlich über dem Molmassen-Permeabilitätslimit für die pflanzliche Zellwand, das heißt, mit Molmassen über 20 kD, zu wählen, und Veränderungen des Salzgehaltes mit Veränderungen des Gehaltes an Ethanol, Aceton cder einer ähnlichen wasserlöslichen organischen Flüssigkeit für das Einführen und Einschließen der Dextranmoleküle kombiniert auszunutzen. Die Molmassenobergrenze einzuschließender hydrophiler Polyelektrolyte mit flexibler Molekülkonfiguration wie Nucleinsäuren, Polygalacturonsäure, Polyacrylsäure und Pektin für den Durchtritt durch dünne negativ geladene Ultrafiltermembranen ist in unerwartetem Ausmaß von der Ionenstärke abhängig, so daß Stoffe mit normalerweise weit über der Permeabilitätsgrenze liegender effektiver Molekülgröße durch Erhöhung der Ionenstärke für die Hohlträgermembran permeabel gemacht und durch anschließende Verringerung der Ionenstärke eingeschlossen werden können.

In ähnlicher Weise ist es möglich, pH-Veränderungen auszunutzen, da sich mit zunehmender Dissoziation der ionischen Gruppen die hydrodynamische Teilchengröße eines Polyelektrolyten bekanntlich (vgl. Autorenkollektiv, Strukturuntersuchungen an Biopolymeren mit spektroskopischen und hydrodynamischen Mehtoden, Akademie-Verlag, Berlin, 1976) vergrößert.

Eine für den Einschluß der Makromoleküle notwendige Herabsetzung der Konzentration permeabilitätsvermittelnder Dispersionsmittelkomponenten kann ohne chemische Umsetzung grundsätzlich durch Verdünnen, Dialyse, Elektrolyse oder Verdunsten erfolgen, wobei der Vorteil der letztgenannten drei Möglichkeiten darin besteht, daß sie ohne Verdünnung der Makromoleküle im Hohlträgerinnenraum durchgeführt werden können. Eine andere Möglichkeit zur Herabsetzung der Konzentration permeabilitätsvermittelnder Dispersionsmittelkomponenten besteht in ihrer chemischen Umsetzung. Beispielsweise können die für die Permeation von Polyanionen vermitteln wirkenden Protonen aus der wäßrigen Lösung durch Reaktion mit basisch wirkenden Stoffen entfernt werden.

Eine Variante der Erfindung besteht darin, die eingeführten hydrophilen Makromoleküle nach der Einstellung des Diffusionsgleichgewichtes im Hohlträgerinnenraum aus der permaetionsvermittelnden Flüssigkeit auszufällen. Flüchtige mit Wasser mischbare organische Flüssigkeiten sind als Fällungsmittel hierfür vorteilhaft. Anschließend an die Fällung der Makromoleküle kann das außerhalb der Hohlträger gebildete Koagulat auf einem Filtertuch abgewaschen und eine Lösungsmitteltrocknung oder Gefriertrocknung der Hohlträger durchgeführt werden. Überführt man das Trockenprodukt in eine permationsverhindernde Flüssigkeit, normalerweise Wasser oder eine wäßrige Salzlösung, kommt es zur Membrantrennung und zum gewünschten Einschluß der Makromoleküle.

Es ist vorteilhaft, die Hohlträger für den erfindungsgemäßen Einschluß von Makromolekülen in der Form eines chromatographischen Bettes anzuordnen. Dabei werden sie vor oder bei der Auftragung einzuschließender Makromoleküle vollständig oder teilweise mit einer permeabilitätsvermittelnden Lösung gesättigt. Wegen des verhältnismäßig geringen äußeren Flüssigkeitsvolumens kann so ein hoher Prozentsatz der eingesetzten Makromoleküle in den Innenraum der Träger eindringen. Anstelle eines feuchten Festbettes kann ein trockenes chromatographisches Bett gemäß Patent DE 3616219 A1 eingesetzt werden. Sättigt man das trockene Festbett mit der Dispersion der einzuschließenden Makromoleküle im permeabilitätsvermittelnden Dispersionsmittel, gelingt die Einführung der Makromoleküle in den Hohlträgerinnenraum ohne Verdünnung.

Die Aufteilung des chromatographischen Bettes in mehrere Böden, in denen Diffusion und Einschluß erfolgen, ermöglicht den Einschluß eines großen Teils der eingesetzten Makromoleküle.

Beim reversiblen Einschluß und bei der anschließenden Freisetzung der Makromoleküle ist, wenn sie polydispers sind, eine Fraktionierung nach der Größe möglich. Beim bisher erläuterten Einschluß werden nur solche Moleküle im Innenraum des Hohlträgers festgehalten, deren Größe im Bereich zwischen den Permeabilitätsgrenzen der Ultrafiltermembran vor und nach der Veränderung des Dispersionsmittels liegt. Der Innenraum mit den eingeschlossenen Makromolekülen kann als Retentatraum, der Zwischenraum zwischen den Hohlträgern als Permeatraum genutzt werden. Der Permeatraum wird mit Elutionsmittel durchströmt, wobei die Zusammensetzung des mobilen Dispersionsmittels unter Ausnutzung bekannter Gesetzmäßigkeiten schrittweise oder kontinuierlich so geändert wid, daß sich die Molekül- oder Teilchengröße der hochmolekularen Stoffe schrittweise oder kontinuierlich im Verhältnis zur Trenngrenze der Membran verringert. Für eine erfolgreiche erfindungsgemäße Größenfraktionierung ist wesentlich, daß die Trenngrenze der Membran in bestimmten Dispersionsmitteln im Dispersitätsgebiet der Makromoleküle liegt, und daß anfangs ein Elutionsmittel eingesetzt wird, in dem die effektiven Molekülgrößen der zu fraktionierenden Stoffe deutlich über ihrenm Minimum und zum Teil über der Trenngrenze der Membran liegen. Die Herabsetzung der Molekülgröße relativ zur Trenngrenze der Membran erfolgt durch Veränderung der Zusammensetzung des Elutionsmittels in mehreren Stufen oder in einem kontinuierlichen Grandienten, z.B. durch Erhöhung der Ionenstärke oder durch Zusatz mit Wasser mischbarer organischer Flüssigkeiten zum wäßrigen Dispersionsmittel, sinngemäß wie oben beschrieben. Wesentlich für die hier dargestellte Fraktionierung ist, daß sie durch Veränderung der Dispersionsmitteleigenschaften und hierdurch verändertes Momassen-Permeabilitätslimits der Hohlträgermembran im Verlauf eines Elutionsprozesses erreicht wird.

Überraschend zeigt sich, daß bei der Elution von in vesikulären Trägern eingeschlossenen polydispersen Dextranen oder Nucleinsäuren in einem Stufengrandienten des Elutionsmittels bei ansteigendem Ethanol-oder $MgCl_2$-Gehalt scharf abgegrenzte Fraktionen mit unterschiedlichem Dispersitätsgrad nachweisbar sind, wobei die Zahl der Fraktionen mit der Zahl der Stufen übereinstimmt. Ein Vorteil des Verfahrens besteht darin, daß die Fraktionen vollständig voneinander getrennt werden können, da nach jedem Increment der Konzentration des die effektive Teilchengröße herabsetzenden Zusatzes eine zeitlich begrenzte Elution in Form eines Peaks erfolgt.

Beispiel 1.

Einschluß eines neutralen hydrophilen Polymers durch Veränderung der Ethanolkonzentration beim Duchlaufen eines chromatographischen Bettes aus vesikulären Trägern

Ein zylindrisches chromatographisches Bett aus salzfreien, getrockneten vesikulären Trägern gemäß DD-Aktenz. G 01 N/317 042 8 von 3 cm Länge und 1,5 cm Durchmesser wurde mit Filterpapier bedeckt und mit 1 ml einer salzfreien wäßrigen Lösung von Ethanol (30 Volumenprozent) und Dextran M 70 (Hersteller VEB Serumwerk Dessau, DDR) in einer Konzentration von 20, mg/ml einseitig befeuchtet. Nach dem Einsaugen der ethanolhaltigen Dextranlösung wurde eine 100 mM Phosphatpufferlösung nach Soerensen bis zur vollständigen Sättigung des selbstsaugendenFestbettes eingeführt. Nach einer Diffusionszeit von 12 h wurde die feuchte Masse der vesikulären Träger mit 20 ml der Phosphatpufferlösung, anschließend mit 20 ml 30%igem Ethanol gewaschen. Die Menge des abgewaschenen Dextrans wurde mit einem Polarimeter 141 M, Firma Perkin & Elmer, erfaßt. In allen Versuchen betrug der eingeschlossene (mit Ethanol-Lösung auswaschbare) Teil des Dextrans mehr als 30 %.

Beispiel 2.

Einschluß eines neutralen hydrophilen Polymers durch Fällung und Trocknung aus Ethanol mit anschließender Quellung in enthanolfreier wäßiger Lösung
5 ml einer 2%igen enthanolhaltigen Dextranlösung (30 Volumenprozent Ethanol, 20 mg/ml Dextran M 70) wurden auf ein mit salzfreier Lösung von Ethanol in Wasser (30 Volumenprozent) gesättigtes, gut verdichtetes chromatographisches Bett aus vesikulärem Trägermaterial (Bettvolumen 6 ml) aufgetragen. Das Einsaugen der Probe erfolgt mit einer Flußrate von 0,1 ml cm$^{-2}$ min$^{-1}$. Nach dem Eindringen der Probe wird die feuchte Trägermasse aus dem Chromatographierohr entnommen und mit 20 mg NaCl (ohne Wasser) verrührt. Nach 30 min wird die Masse in kleinen Portionen unter starkem Rühren in ein Becherglas mit 30 ml 96%igem Ethanol übertragen. Die Träger werden auf einem runden Filtertuch aus Polyamidseide mit 0,1 mm Porendurchmesser und 3 cm Durchmesser abgefiltert, zur Entfernung des Restwassers mit 10 ml Ethanol in gepackter Form gewaschen, scharf abgesaugt und im Rotationsverdampfer unter Zusatz von 0,3 ml n-Butanol getrocknet. Das getrocknete Pulver wird mit 10 ml dest. Wasser gequollen und im Chromatographierohr mit Aqua dest. bis zu einer Gesamtmenge des Eluats (Eluat 1) von 20 ml gewaschen. Der Dextrangehalt des so erhaltenen Eluats 1 wird am Polarimeter 141 M gemessen. Anschließend wird nochmals mit 10 ml Aqua des, gewaschen, wobei das Eluat (Eluat 2) zur polarimetrischen Untersuchung aufgefangen wird. Zuletzt wird die Packung mit 20 ml salzfreier wäßriger Ethanol-Lösung (30 Volumenprozent) eluiert, das Eluat (Eluat 3) wird aufgefangen. In allen Versuchen beträgt der Anteil des eingeschlossenen Dextrans (Eluat 3) an der gesamten im Trockenprodukt vorhandenen Menge mehr als 50 % und mehr als 20 % der insgesamt eingesetzten Menge. Eluat 2 enthielt keine signifikante Dextranmenge. Zu einem entsprechenden Ergebnis kommt man, wenn anstelle von Dextran M 70 eine aus diesem Stoff und einem niedermolekularen Stoff zusammengesetzte Verbindung (beispielsweise Blue Dextran) oder ein durch Peiodatoxidation aktiviertes Dextran eingesetzt wird.

Beispiel 3.

Einschluß von Polyelektrolyten durch Veränderung der Ionenstärke beim Durchlauf durch ein chromatographisches Bett aus vesikulären Trägern Gemäß Patent DD 247 570 A3 hergestellte und getrocknete vesikuläre Träger werden in einem 200 mM MgCl$_2$ enthaltenden Puffer (10 mM Tris-HCl, pH 7,5) suspendiert. Eine Packung dieser Träger in einer Chromatographie-Säule (Bettvolumen 5 ml, Innendurchmesser 16 mm) wird mit Filterpapier bedeckt. Die Säule wird mit einem registrierenden UV-Detektor mit Durchflußküvette (Wellenlänge 254 nm), einer Durchfluß- Leitfähigkeitsmeßzelle und einem Fraktionssammler verbunden. Das Bett wird solange mit dem 200 mM MgCl$_2$ enthaltenden Puffer gespült, bis das Eluat von UV absorbierenden Stoffen frei ist. Anschließend erfolgt der Wechsel zu einem Elutionspuffer, der 20 mM MgCl$_2$ enthält. Mit diesem wird gewaschen, bis die Leitfähigkeit des Eluats gleich der des reienen Puffers ist. 50 μl einer Lösung von degradierter Ribonucleinsäure (RNA aus Hefe, Boehringer Mannheim, 1 mg/ml in 10 mM Tris-HCl, pH 7,5) werden auf die Säule aufgebracht und nach Einsaugen der Probe in kleinen Portionen mit magnesiumchloridfreiem Puffer gespült. Infolge des Pufferwechsels bleibt ein Teil der Nucleinsäuremoleküle (20 bis 30 Prozent) im Innenraum der Träger eingeschlossen und kann diesen vorerst nicht verlassen. Die nicht eingeschlossenen Moleküle werden eluiert.

Beispiel 4.

Fraktionierung von Polyanionen an vesikulärem Trägermaterial Nucleinsäure werden in vesikulären Trägern entsprechend dem in Beispiel 3 dargestellten Protokoll eingeschlossen. Anschließend wird die Säule mit dem Trispuffern unterschiedlichen Mg Cl$_2$ - Gehaltes gespült. Nach Spülen der Säule mit magnesiumchloridfreiem Puffer (mindestens 2 Säulenvolumen bzw. 10 ml) erfolgt die fraktionierte Freisetzung der eingeschlossenen Moleküle durch Puffer mit stufenweise ansteigender Magnesiumchloridkonzentration:
0 bis 40 ml 0,2 mM
40 bis 70 ml 2,0 mM
70 bis 80 ml 20 mM
Anhand der Extinktion bei 254 nm können deutlich drei verschiedene, scharf abgegrenzte Fraktionen detektiert werden.
Abb. 1 zeigt die schrittweise Elution der zurückgehaltenen (eingeschlossenen) Moleküle einer RNA-Probe durch Puffer ansteigender MgCl$_2$-Konzentration. Probe: RNA (aus Hefe Boehringer Mannheim, 1 mg/ml in 10 mM Tris-HCl, pH 7,5, Probenvolumen: 50 μl, Säule: 16 x 23 mm
In entsprechender Weise wurden darüber hinaus mit vergleichbarem Ergebnis fraktioniert:
- degradierte Desoxyribonucleinsäure aus Heringssperma (SERVA, Heidelberg)
- degradierte Polygalakturonsäure aus Citrus (SERVA, Heidelberg)

Beispiel 5.

Fraktionierung von Dextran an vesikulärem Trägermaterial Eine analog Beispiel 1 hergestellte Chromatographiesäule mit vesikulärem Trägermaterial wird bis zur Ionenfreiheit des Eluats (konduktometrische Kontrolle) mit einer Ethamnol-Wasser-Mischung (35 Volumenprozent Ethanol) eluiert. Anschließend wird ein Volumen von 0,1 ml einer Lösung eines polydispersen Dextrans in Phosphatpuffer (50 mg/ml, 0,1 M Phosphatpuffer, pH 6,5 unter Zusatz von 0,05.% Natriumazid, Dextran T 250 der Fa. Pharmacia, Schweden) aufgetragen und nach zweimaligem Nachspülen mit jeweils 0,1 ml des Phosphatpuffers mit einer größeren Phosphatpuffermenge unter polarimetrischer Kontrolle eluiert, bis im Eluat kein Dextran mehr nachgewiesen wird. Ein Teil des aufgebrachten Dextrans (etwa 20 %) wird dabei durch den Wechsel der Zusammensetzung des Elutionsmittels im Innenraum de Zellwandvesikeln eingeschlossen und kann nicht eluiert werden. Im Anschluß daran erfolgt die Elution in mehreren Stufen, zunächst mit dem verwendeten Phosphatpuffer unter Zusatz von 25 Volumenprozent Ethanol, wobei die erste Fraktion eluiert wird, dann mit einer Ethanol-Wasser-Mischung (30 Volumenprozent Ethanol), wobei eine Fraktion mit mittlerer Molekülgröße eluiert wird, zuletzt mit einer Mischung aus Ethanol und Wasser (35 % Ethanol), die zur vollständigen Elution (eingeschlossene Dextranmoleküle mit dem größten Molekulargewicht) führt.

Beispiel 6

Einschluß einer Polyethylenglykol-Flüssigphase in veskulären Trägern Die vesikulären Träger gemäß Beispiel 1 werden mit einer Mischung aus Ethanol und Wasser (19/1) gesättigt. Die ethanolgesättigten vesikulären Träger werden mit Polyethylenglykol (PEG) mit einer mttleren Molmasse von 20 kD (SERVA) versetzt (0.15 g/ml des gepackten Volumens). Nach der Auflösung des PEG wird die ethanolfeuchte Masse auf einer G 2 - Fritte scharf abgesaugt und so von der frei beweglichen ethanolischen PEG-Lösung befreit. Anschließend wird die nun körnige ethanolfeuchte Masse mit einem 1,5 M Kaliumphosphat-Puffer , pH 7 versetzt. Es bilden sich in den Vesikelhohlräumen Tropfen einer PEG-Entmischungsphase. Die außerhalb der vesikulären Partikeln entstandenen Tröpfchen können mit dem Puffer abgewaschen werden. Bringt man die so präparierten vesikulären Träger in Wasser, lösen sich die Tröpfchen auf, ohne daß sich das PEG in der Außenphase verdünnen kann. Die Tröpfchen bilden sich wieder, wenn das präparierte Material wieder in den 1,5 M Phosphatpuffer überführt wird. Die mit PEG gefüllten vesikulären Träger sind in Wasser turgeszent. Das eingeschlossene PEG bewirkt eine verringerte Kompressibilität der Partikelmasse durch osmotisch bedingten Binnendruck der Vesikeln. Mit den präparierten Vesikeln kann eine Kombination von Ausschluß- und Verteilungschromatographie durchgeführt werden, wobei die eingeschlossene PEG-Entmischungsphase zahlreiche permeable wasserlösliche Komponenten, besonders solche mit hydrophoben funktionellen Gruppen und Anionen anreichert bzw. chromatographisch verzögert.

**Ansprüche**

1. Verfahren zur Herstellung von Hohlträgern mit eingeschlossenen, diffusionsfähigen hochmolekularen Stoffen mit möglicher Größenfraktionierung von Makromolekülen, dadurch gekennzeichnet , daß Hohlträger, begrenzt von einer geschlossenen, semipermeablen Ultrafiltermembran, mit einzuschließenden diffusionsfähigen hochmolekularen polymeren Stoffen in einem permeationsvermittelnden Dispersionsmittel, in dem das Molmassen-Permeabilitätslimit der Membran für die einzuschließenden polymeren Stoffes höher liegt als deren Molmasse, dispergiert, danach das permeabilitätsvermittelnde Dispersionsmittel gegen ein anderes ausgetauscht oder in seiner chemischen Zusammensetzung verändert wird, wobei der Austausch oder die Veränderung des Dispersionsmittels unter Ausnutzung bekannter Gesetzmäßigkeiten über die Dispersionsmittelabhängigkeit der Molekülgröße im Verhältnis zur Durchlässigkeit der Ultrafiltermembran so durchgeführt wird, daß das Molmassen-Permeabilitätslimit der Membran für die einzuschließenden Makromoleküle unter die Molmasse der einzuschließenden Makromoleküle herabgesetzt wird bzw. ein permeabilitätsverhinderndes Dispersionsmittel für die einzuschließenden Makromoleküle vorliegt, und daß die Hohlträger im permeabilitätsverhindernden Dispersionsmittel verbleiben oder anschließend mit einem permeabilitätsvermittelnden in Verbindung gebracht werden.

2. Verfahren nach Anspruch 1, dadurch gekennzeichnet , daß als Hohlträger bekannte vesikuläre Träger mit aus einer Pflanzenzellwand bestehenden oder aus ihr hervorgegangenen Ultrafiltermembran eingesetzt werden.

3. Verfahren nach Anspruch 1 bis 2, dadurch gekennzeichnet , daß als einzuschließende hochmolekulare Stoffe hydrophile Polymere mit stark hydratationsabhängiger Molmassen-Permeabilitätsgrenze wie Dextrane, weitere wasserlösliche neutrale oder saure Kohlenhydrate, Nukleinsäuren, Polyethylenglycol, Polyvinylpyrrolidon, Polyacrylamid, Polyacrylsäure oder Verbindungen anderer Stoffe mit diesen polymeren Stoffen eingesetzt werden und als permeabilitätsvermittelndes Dispersionsmittel eine wasserlösliche organische Flüssigkeit oder eine Mischung aus Wasser und einer als permeationsvermittelnde Komponente wirkenden wasserlöslichen organischen Flüssigkeit mit einem unterhalb der Fällungskonzentration für die Makromoleküle liegenden Mengenanteil der organischen Flüssigkeit, eingesetzt und die Verringerung des Mengenanteils der permeabilitätsvermittelnden organischen Komponente im Dispersionsmittel auf dem Wege der Verdünnung, der Dialyse oder des Verdampfens durchgeführt wird.

4. Verfahren nach Anspruch 3, dadurch gekennzeichnet, daß als einzuschließende Makromoleküle Dextrane oder Dextranderivate mit einer Molekulargewichtsverteilung im Größenbereich über 20 kD, als permationsvermittelndes Dispersionsmittel eine salzfreie oder salzarme wäßrige Lösung von Aceton oder Ethanol mit einem Ethanol- oder Acetongehalt wenig unterhalb der Fällungskonzentration für die Dextrane oder Dextranverbindungen eingesetzt und danach eine Erhöhung der Salzkonzentration und/oder Verringerung der Ethanol- bzw. Acetonkonzentration im Dispersionsmittel vorgenommen wird.

5. Verfahren nach Anspruch 1 bis 2, dadurch gekennzeichnet , daß als permeationsvermittelndes Dispersionsmittel für einzuschließende hydrophile Polyanionen mit stark ionenstärkeabhängiger Molmassen-Permeabilitätsgrenze wie Nucleinsäuren, Pektin, Polygalacturonsäure, Polyacrylsäure oder Verbindungen derartiger Polyanionen mit anderen Stoffen eine wäßrige Salzlösung mit einer Salzkonzentration unter der Fällungskonzentration für die einzuschließenden Polyelektrolyte eingesetzt und anschließend eine Herabsetzung der Ionenstärke des wäßrigen, anfangs permeationsvermittelnden, Dispersionsmittels auf dem Wege der Verdünnung, der Dialyse oder der Elektrolyse vorgenommen wird.

6. Verfahren nach Anspruch 1 bis 5, dadurch gekennzeichnet , daß als einzuschließende hochmolekulare Stoffe Polymere mit bekannten kupplungsfähigen Gruppen für die Bindung von Wirkstoffen und Enzymen oder mit gebundenen Rezeptoren für eine Affinitätsbindung eingesetzt werden.

7. Verfahren nach Anspruch 6, dadurch gekennzeichnet , daß als einzuschließender hochmolekularer Stoff ein mit Periodat teilweise oxidiertes hochmolekulares Dextran eingesetzt wird.

8. Verfahren nach Anspruch 1 bis 7, dadurch gekennzeichnet , daß die Hohlträger nach dem Zusatz des permeabilitätsverhindernden Dispersionsmittels oder der permeabilitätsverhindernden Dispersionmittelkomponenten auf einem Sieb oder einer Fritte mit Dispersionsmittel für die eingeschlossenen Makromoleküle eluiert werden und dabei durch stufenweise oder kontinuierliche Veränderung der chemischen Zusammensetzung des Elutionsmittels das Molmassen-Permeabilitätslimit der Makromoleküle in an sich bekannter Weise wieder heraufgesetzt wird.

9. Verfahren nach Anspruch 8, dadurch gekennzeichnet , daß als permebilitätsvermittelnde Komponente eine mit Wasser mischbare organische Flüssigkeit eingesetzt wird.

10. Verfahren nach Anspruch 8, dadurch gekennzeichnet , daß als permeationsvermittelnde Komponente ein Salz eingesetzt wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 603 685 (SLEYTR et al.) * Seite 1, Zeilen 13-14; Seite 3, Zeilen 1-23; Seite 4, Zeilen 1-8; Seite 8, Zeilen 23-26; Seite 9, Zeilen 20-24; Seite 10, Zeilen 26-32; Seite 13, Zeilen 14-33; Seite 14, Zeilen 1-28 * --- | 1,2,3, 10 | B 01 D 69/02 |
| A | WO-A-8 806 442 (THE LIPOSOME CO.) * Seite 7, Zeilen 27-33; Seite 11, Zeilen 32-37; Seite 14, Zeilen 19-36; Seite 16, Zeilen 16-37; Seite 17, Zeilen 1-15; Seite 54, Zeilen 23-32 * --- | 1,9,10 | |
| A | WO-A-8 404 932 (WASHINGTON RESEARCH FOUNDATION) * Seite 2, Zeilen 1-13 * ----- | 1 | |

|  | | | RECHERCHIERTE SACHGEBIETE (Int. Cl.5) |
|---|---|---|---|
| | | | B 01 D A 61 K |

Der vorliegende Recherchenbericht wurde für alle Patentansprüche erstellt

| Recherchenort | Abschlußdatum der Recherche | Prüfer |
|---|---|---|
| DEN HAAG | 18-09-1990 | KERRES P.M.G. |